# EUROPEAN PATENT SPECIFICATION

(11) **EP 1 450 884 B1**
(45) Date of publication and mention of the grant of the patent: **21.04.2010**
(21) Application number: 01970819.7
(22) Date of filing: 13.09.2001
(51) Int. Cl.: A61M 5/178, A61F 9/00, A61F 9/007

(54) **Medical device for Retina operations**
medizinisches Instrument für Retina Operationen
Dispositif medical pour operation de la rétine

(43) Date of publication of application: 01.09.2004
(73) Proprietor: Weiss, Jeffrey N., Parkland, FL 33067 (US)
(72) Inventor: Weiss, Jeffrey N., Parkland, FL 33067 (US)
(74) Representative: Hall, Robert Leonard
(86) International application number: PCT/US2001/028426
(87) International publication number: WO 2003/022336

(56) References cited:
- US-A- 4 212 297
- US-A- 5 792 099

## Description

### BACKGROUND OF THE INVENTION

### 1. Field of the Invention

This invention relates to medical diagnostic and therapeutic apparatus and, in particular, to apparatus for cannulating a retinal blood vessel such that a medication may be injected or a quantity of fluid removed from the blood vessel. Alternatively, a catheter, wire or stent may be placed through the cannula to treat or diagnose an area remote from the insertion site.

### 2. Description of Related Art

The cannulation of a retinal blood vessel is difficult as the lumen of the blood vessels is less than 200 µm in size. The present day ocular instruments are too large to cannulate the vessel and the dexterity required to maintain the cannula within the blood vessel for several minutes is not commonly available. The piercing of a blood vessel elsewhere in the body to inject medications, perform surgical procedures or remove blood for analysis and treatment is commonly performed.

US patent US-A-5792099 (decamp et al.) describes an ocular instrument comprising a cannula and a syringe containing viscoelastic material. The DeCamp device is typically used to deliver viscoelastic material to fill the chambers of the eye in order to protect sensitive tissues during surgery.

It is therefore, to the effective resolution of the aforementioned problems and shortcomings that the present invention is directed.

Accordingly, it is an object of this invention to provide a microcannula or micropipette whose lumen is small enough to be safely placed within the lumen of a retinal blood vessel and by its configuration is parallel to the lumen when placed through a standard sclerotomy site, as commonly used in vitreoretinal surgery. The terms microcannula and micropipette are used interchangeably throughout the application. To the extent that such terms differ in any way in meaning, if any, then the broadest definition of the two terms is considered to be the definition for both terms for purposes of the instant invention disclosure.

It is another object of this invention to provide, by its configuration and method of attachment, a stable support such that the micropipette may be securely held within the blood vessel so that subsequent maneuvers may be safely accomplished.

It is still another object of this invention to provide a micromanipulator such that the micropipette may be remotely advanced to perforate the retinal blood vessel.

It is yet another object of this invention to provide a portable device that may be easily attached to a standard operating surgical wrist rest and is stable in its "X", "Y" and "Z" planes.

It is a further object of this invention to provide a device that, by its configuration and method of attachment, does not inhibit the surgeon's view when using an operating microscope or otherwise interfere with the use of the operating microscope. It is yet another object of this invention to provide a safe method such that the surgical procedure may be performed.

### BRIEF SUMMARY OF THE INVENTION

The present invention is defined in the appended claims.

### BRIEF DESCRIPTION OF THE SEVERAL VIEWS OF THE DRAWINGS

The invention may be better understood by reference to the drawings in which:
Figure 1 is a front view of a first embodiment for the micropipette (microcannula) of the present invention;
Figure 2 is a front view of a second embodiment of the micropipette in which an illumination member such as a fiber optic light source is attached to the side of the micropipette to provide illumination during the operation;
Figure 3 is a front view of a third embodiment of the micropipette wherein the micropipette and fiber optic are enclosed within a protective sheath or tube to minimize breakage when placed into the eye, the protective sheath or tube can also be used for a microcannula without a fiber optic;
Figure 4 is a perspective view of the preferred embodiment for the micropipette, micromanipulator, positioner and base of the present invention attached to a conventional wrist rest;
Figure 5 is another perspective view of the preferred embodiment for the micropipette, micromanipulator, positioner and base of the present invention;
Figure 6 illustrates a view of the micropipette when placed through the sclerotomy site into the eye;
Figure 7 illustrates the tip of the micropipette overlying and parallel to the retinal blood vessel to be cannulated;
Figure 8 is a perspective view of the micropipette when placed through the sclerotomy site into the eye;
Figure 9 illustrates a front view of the fourth embodiment of the microcannula in which the shaft of the microcannula is enclosed by a protective sheath;
Figure 10 illustrates a front view of the fifth embodiment of the microcannula in which the shaft of the microcannula is enclosed within a protective sheath with a cover that retracts and exposes the tip of the microcannula;
Figure 11 illustrates a front view of the sixth embodiment of the microcannula in which the protective sheath may be retracted thus exposing the microcannula tip;
Figure 12 illustrates a front view of the seventh embodiment of the microcannula in which the device is encased in a case. The protective sheath may be retracted thus exposing the microcannula tip. An illumination member, such as a fiber optic or other light source may be attached to the microcannula assembly such that the tip of the microcannula or the area surrounding the tip of the microcannula may be illuminated;
Figure 13 illustrates a front view of the eighth embodiment of the microcannula where the protective sheath may also be substantially sharp;
Figure 14 is a perspective view of the preferred embodiment for the microcannula, clamp with stabilization post, stabilization arm and illumination positioner attached to a conventional surgeons wrist-rest; and
Figure 15 illustrates the illumination positioning arm that attaches to the stabilization post and an accessory arm that contains a series of openings in which to place a fiber optic thus directing the angle of the illumination within the eye.

### DETAILED DESCRIPTION OF THE INVENTION

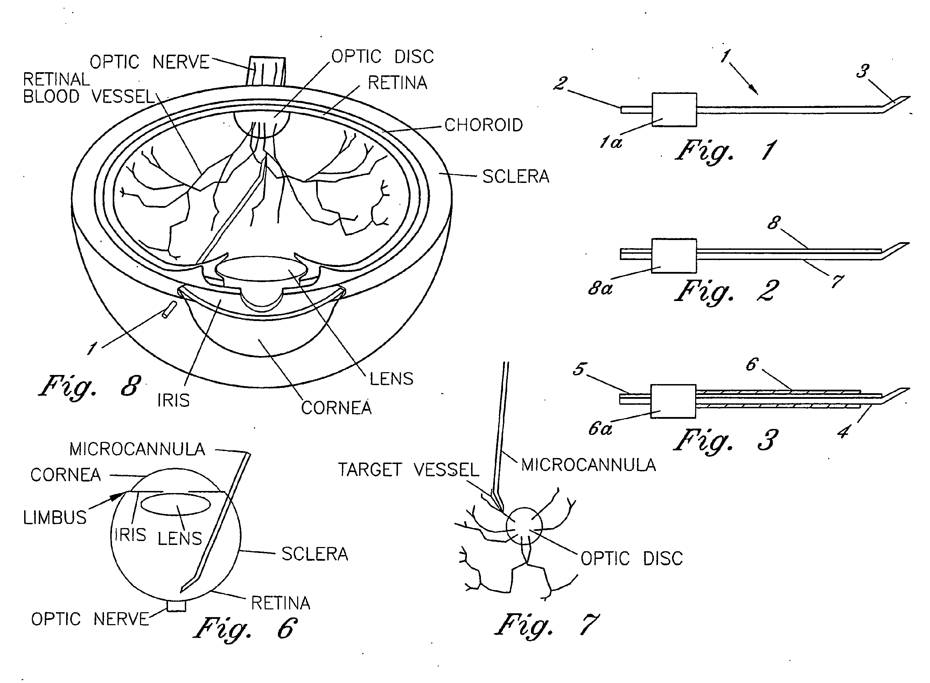

Figure 1 illustrates a first embodiment for the micropipette/microcannula (1) showing the opening (2) that is preferably connected to a surgical tubing and the tip (3) of the micropipette oriented at an approximately 135 degree angle, although other ranges are possible. Tip (3) is angled so that it may safely cannulate the retinal vessel when micropipette (1) is placed through a standard retinal surgical sclerotomy site. While glass is suggested for the material because of its ease of fashioning, strength, transparency, etc., other materials may be used. It is essential, however, that the materials maintain substantial strength when fashioned to perform retinal vessel cannulation. A handle (1a) is shown attached to the body member of micropipette (1). Handle (1a) fits securely within la micropipette holder (10) by inserting the end of micropipette (1) associated with handle (1a) and handle (1a) into the front opening of holder (10). Once inserted micropipette (1) is held in place by a setscrew associated with the holder (10).

As seen in Figure 2, an alternative embodiment of the micropipette/microcannula is illustrated. In this embodiment, a fiber optic (8) is attached to the micropipette body (7) to provide illumination such that an illuminated infusion cannula is not required. If a vitrectomy is not performed then one sclerotomy for the micropipette and fiber optic is all that is necessary. A handle (8a) is provided and fits securely within the holder (10) and is held in place by a set screw within the holder (10), similar to as described for micropipette (1).

Figure 3 illustrates a further alternative embodiment for the micropipette (4) where a fiber optic for illumination is included (5) and both items are placed within a tube or needle (6). The purpose of the tube or needle is to protect the enclosed instruments such that they may be safely inserted through the sclerotomy site without breakage. Both the fiber optic and the micropipette ends are at the end or protrude from the end of the tube or needle. The micropipette and fiber optic may be advanced through the end of the tube or needle once it has been placed within the eye. A handle (6a) is illustrated that fits securely within the holder (10) and may be firmly held in place by a set screw or locking mechanism within the holder (10), as previously described above. If a vitrectomy is not performed then one sclerotomy for this device is all that is necessary.

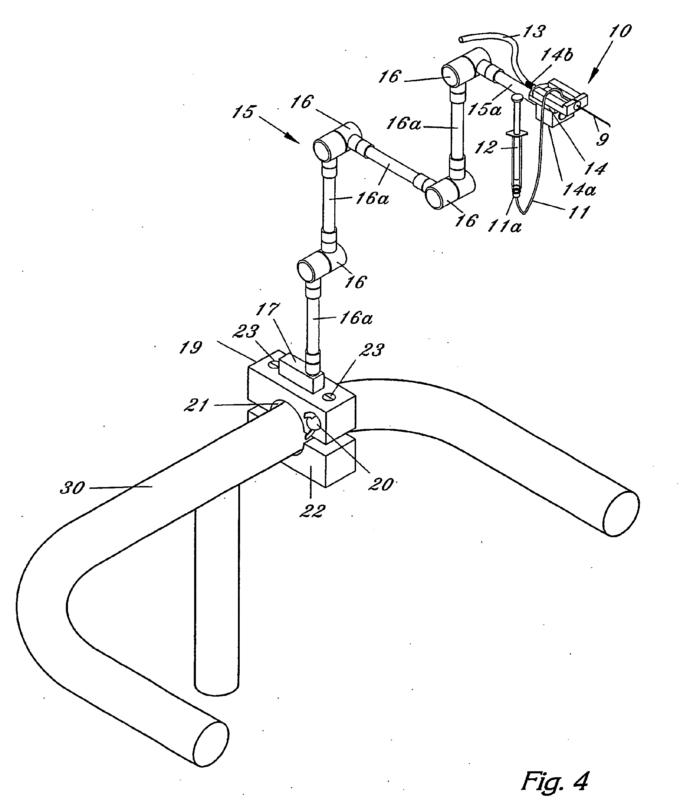

Figure 4 illustrates the micropipette (9) attached to the holder (10). A screw handle (14b), which controls the position of the holder (10), is attached to a flexible tube (13) so the micromanipulator may remotely advance the micropipette. Screw handle (14b) is associated with a micromanipulator (14). Preferably, screw handle (14b) is connected to micromanipulator (14). Holder (10) is attached to the micromanipulator. In one embodiment, the micromanipulator is a miniature translation stage, using dual dowel pin bearings. One such micromanipulator is made by the Newport Corporation located in Irvine, California. The Newport micromanipulator has a stage, which has a range of travel of approximately four (4mm) millimeters.

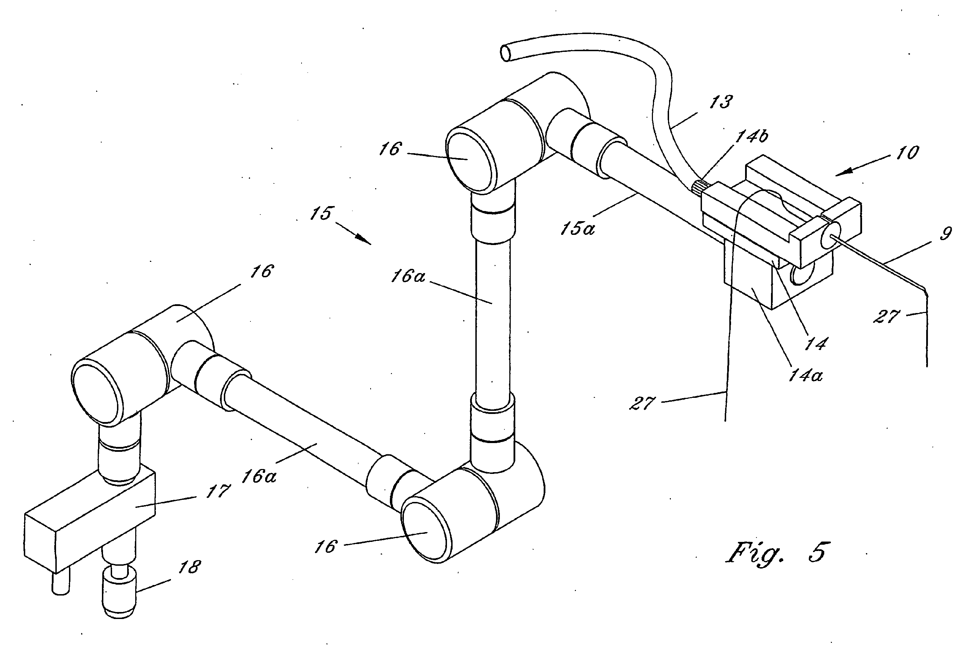

In one embodiment (Figure 4), the non-tip end of the micropipette is preferably attached to standard surgical tubing (11). The tubing (11) is attached to a connector (11a), which is connected to a syringe (12) that is used to inject medication or withdraw fluid from the retinal blood vessel. In certain situations medication such as t-PA can be injected into the retinal vessel. Alternatively, a dye can be injected into the retinal vessel for diagnosing purposes. Alternatively, a catheter, wire or stent (27) may be advanced through the microcannula for diagnosing, testing or treatment of an area located at a distance from the insertion site (Figure 5).

It should also be apparent to those skilled in the art that a foot pedal or other switch may control (i.e. electrically, pneumatically, mechanically, etc.) the micromanipulator and injector or withdrawing device so it may be activated by the surgeon. These alternative embodiments are considered within the scope of the invention.

The micromanipulator (14) is attached to a base (14a) which is attached to a positioner (15) that is freely mobile in the "X", "Y" and "Z" planes due to the multiplicity of joints (16), connected by elongated members (15a and 16a). It would be apparent to those skilled in the art that the positioner may also be electrically controlled by servo-motors and activated by the surgeon with a foot pedal or other switch. Such alternatives are also considered within the scope of the invention. Positioner (15) is not limited to any specific amount of elongated members.

The positioner can be attached to a base (17). In one embodiment, an attachment post (18) fits into a hole within another base (19). Preferably, set screws or wing nuts (20), are provided, on either side of the base which is used to secure the post to the base. In order to make the base secure, base (19) attaches to another base (22) by two screws (23). Base (19) fists above the standard ophthalmic surgical wrist rest (30) which is oriented perpendicular to bases (19) and (22). The wrist rest fits within the hole (21) that exists between bases (19) and (22). Base portion (22) completes the base and is located underneath the wrist rest. Alternatively, the positioned may be attached directly to the wrist rest or connected to the operating microscope or operating table. Additionally, the bases can be sized to fit other objects in the operating room. Changes in modifications within the spirit and scope of the invention will be apparent to those skilled in the art. Such modifications and changes are intended to be covered by the claims herein.

As seen in. Figures 6 through 8, a sclerotomy can be made at the standard distance from the limbus and an illuminated infusion cannula can be placed through the sclera at this point. A pars plana vitrectomy may or may not be necessary with further experience. Another or second sclerotomy can be made at the standard distance from the limbus such that the micropipette/microcannula is substantially parallel to the retinal blood vessel chosen to be cannulated. The micropipette is then placed through the sclerotomy overlying the selected retinal blood vessel. The intraocular pressure can be lowered to approximately 5mm of Mercury to allow dilation of the vessel. Once the blood vessel is perforated, it may be advantageous to raise the intraocular pressure to minimize bleeding. The retinal blood vessel may be cannulated manually or the micromanipulator used to advance the micropipette into the retinal blood vessel.

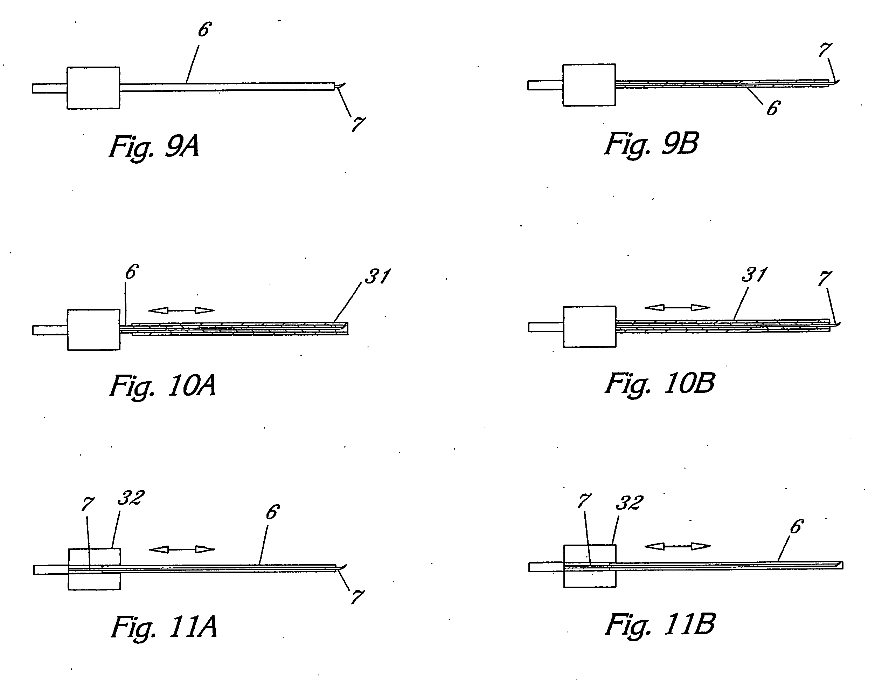

Figures 9A and 9B illustrate a front view of the fourth embodiment of the microcannula in which the shaft (7) of the microcannula is enclosed by a protective sheath (6).

Figures 10A and 10B illustrate a fifth embodiment for the microcannula showing the microcannula (7) within a protective sheath (6) that protects the shaft of the microcannula and protective cover (31) that protects the tip of the microcannula (Figure 10A). The protective sheath and cover may be made of metal, plastic, or other materials that protects the shaft and tip of the microcannula. As seen in Figure 10B, the cover may be retracted once the microcannula is within the eye and is replaced or extended outwards once the procedure is complete and the microcannula is ready for removal from the eye, such that tip breakage is minimized.

Figures 11A and 11B illustrate a front view of the sixth embodiment of the microcannula in which the protective sheath (6) may be retracted into the handle (32) thus exposing the microcannula tip (Figure 11A). Handle (32) also protects an otherwise exposed/unprotected portion of the shaft (7) of the microcannula when the protective member is in an extended/outward position over the beveled tip (Figure 11B). The handle attached to the various microcannulas of the invention, including but not limited to handle (32) can be constructed from various materials such as nylon, plastic, delfin, etc.

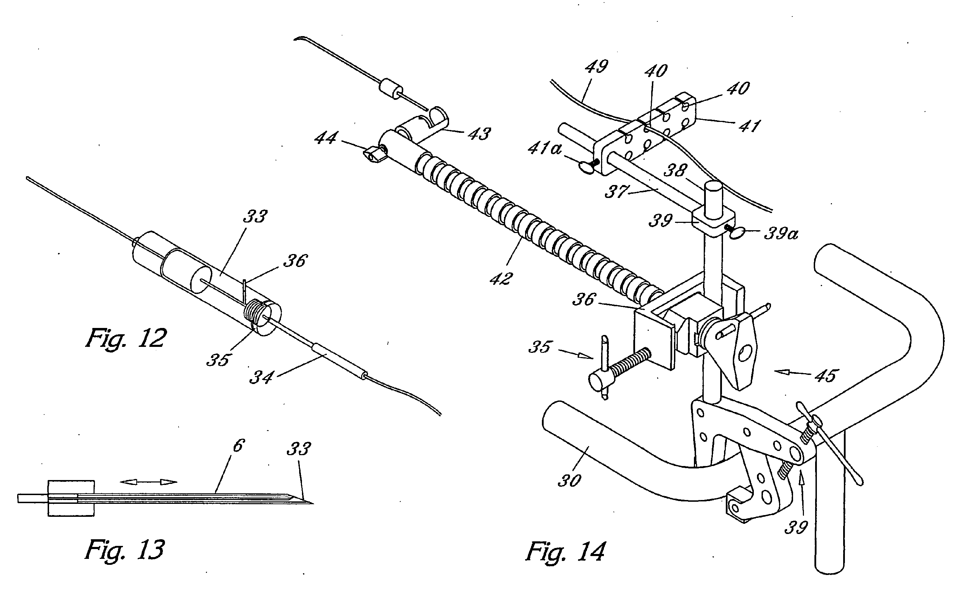

Figure 12 illustrates a seventh embodiment for the microcannula, wherein a portion of the shaft of the microcannula is encased in a hard case (33) which may be made from plastic, metal, or another robust material. An illumination member, such as a fiber optic (34) traditionally used in retinal surgery, or another illumination member or light source, may be attached to the case and secured in place by a gasket assembly (35). The type of light source and method of attachment will determine the size and degree of illumination provided. Alternatively, the light member may be attached within or outside the protective cover. The side port (36) is in communication with a device or tubing that is connected to a syringe or other device that will allow the injection or egress of fluid or other material through the microcannula.

Figure 13 illustrates an alternate embodiment for the microcannula assembly where the tip (33) of the sheath (6) surrounding the microcannula may be sharp enough to perforate the sclera. This embodiment obviates the need for the traditional knife or MVR blade that is generally used by the surgeon to make a hole in the sclera, or sclerotomy, through which the surgeon places instruments into the eye. Once the device is inside the eye, the sheath (6) is retracted exposing the tip of the microcannula and the.procedure is performed.

Figures 14 illustrates a microcannula (i.e. any of the microcannulas disclosed herein) which can be attached to a stabilization arm (42) by a holder (43) and set screw assembly (44) or similar device. The holder (43) includes a clamp-mechanism that allows for different sizes of microcannulas to be retained. The stabilization arm (42) is preferably maneuverable in the x-y-z position and may be connected by another set screw assembly (35) with clamp mechanism (36) or similar device to a stabilization post (38) which can be attached to a clamp (39) that can be attached to a standard surgical wrist-rest (30) or other object. The tension within the stabilization arm is controlled by a tension control assembly (45). The tension along the stabilization arm controls the flexibility of the arm. The stabilization arm may be loosely tightened such that it is sufficiently flexible along its length and allows the microcannula to be easily placed into the eye. Prior to the cannulation of the retinal blood vessel, the tightening of the stabilization arm by the tension control assembly is performed such that the microcannula is steady within the eye, but where small movements are still possible with mild force by the surgeon, if desired. This allows the surgeon to place the microcannula within the blood vessel and allows it to maintain its position within the blood vessel during an infusion of medication, withdrawal of a sample, placement of an instrument, etc. The force required by the surgeon against the stabilization arm dampens any unintended movement by the surgeon such as tremor which may occur during the procedure.

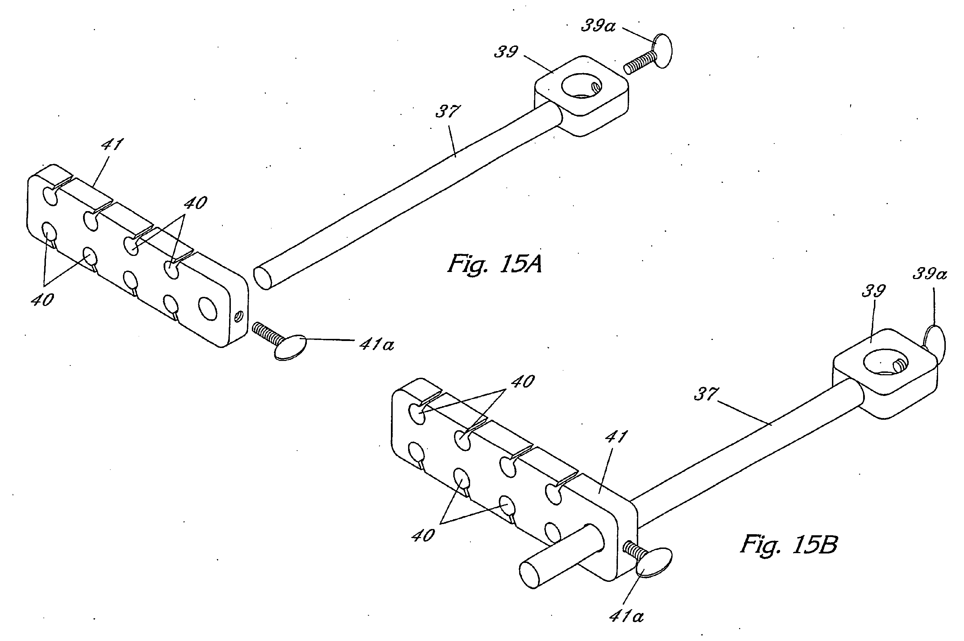

Figures 15A and 15B illustrate the illumination positioning arm (37). As seen in Figure 14, illumination positioning arm (37) attaches to the stabilization post (38) through circular member (39). The arm is held in place by a set screw (39a). One or more, and preferably a series of, openings (40) are placed within an accessory arm (41) that is secured to the illumination positioning arm by a set screw (41a). By setting the angle of the illumination positioning arm in relation to the patient's head, the location of the accessory arm, and the opening in which the fiber optic (49) is placed, the desired location/intensity of illumination within the eye is achieved.

The stabilization arm, stabilization post and illumination positioning arm and accessory arm are preferably made from an easily sterilizable material, such as stainless steel or rubber, though other materials may be used and are considered within the scope of the invention.

In all embodiments, the micropipette/microcannula can be preferably designed to fit a eighteen (18) through twenty (20) gauge sclerotomy site. However, such is not limiting and other gauge sclerotomy sites can be chosen, and the micropipette designed accordingly, and are considered within the scope of the invention.

Though not to be considered limiting, the dimension ranges for the micropipette/microcannula for all embodiments, can preferably consist of the following:
(a) first body portion associated with beveled tip end - length approximately 500-1000 µm;
(b) tip end beveled at approximately twenty (20°)-thirty (30°) degrees;
(c) second body portion associated with handle - length approximately 60 - 100 mm;
(d) beveled tip end - outer diameter approximately 50-100 µm - inner diameter approximately 40-70 µm; and
(e) angle between first body portion and second body portion approximately 100° - 180°, depending on area in which it is used for.

The instant invention has been shown and described herein in what is considered to be the most practical and preferred embodiment. It is recognized, however, that departures may be made therefrom within the scope of the claims and that obvious modifications will occur to a person skilled in the art.

## Claims

1. A microcannula (1,9) for use in cannulating retinal blood vessels, said microcannula comprising:
an elongated hollow body member having an angled first end (3) defining a beveled tip and a second end (2) for connection to a tubing member, said body member having a first body portion associated with said beveled tip and a second body portion associated with said second end; wherein the first end of said body member is suitable for insertion through a retinal surgical sclerotomy site and to cannulate a retinal blood vessel.

2. Apparatus according to claim 1, wherein a handle (1a,6a,8a) is disposed on said body member near said second end.

## Patentansprüche

1. Mikrokanüle (1, 9) zur Verwendung beim Kanülieren retinaler Blutgefäße, wobei die Mikrokanüle aufweist:
ein längliches, hohles Körperteil mit einem abgewinkelten ersten Ende (3), das eine abgeschrägte Spitze definiert, und einem zweiten Ende (2) zur Verbindung mit einem Röhrenteil, wobei das Körperteil einen ersten Körperbereich, der der abgeschrägten Spitze zugeordnet ist, und einen zweiten Körperbereich hat, der dem zweiten Ende zugeordnet ist, wobei das erste Ende des Körperteils zum Einführen durch eine chirurgische retinale Sklerotomiestelle und zum Kanülieren eines retinalen Blutgefäßes geeignet ist.

2. Vorrichtung nach Anspruch 1, wobei am Körperteil nahe am zweiten Ende ein Griff (1a, 6a, 8a) angeordnet ist.

## Revendications

1. - Microcanule (1, 9) destinée à être utilisée dans la canulation de vaisseaux sanguins rétiniens, ladite microcanule comprenant :
un élément de corps creux allongé ayant une première extrémité inclinée (3) définissant une pointe biseautée et
une seconde extrémité (2) pour un raccordement à un élément de tubulure, ledit élément de corps ayant une première partie de corps associée à ladite pointe biseautée et une seconde partie de corps associée à ladite seconde extrémité ; la première extrémité dudit élément de corps étant appropriée pour une introduction à travers un site de sclérotomie chirurgicale rétinienne et pour effectuer la canulation d'un vaisseau sanguin rétinien.

2. - Appareil selon la revendication 1, dans lequel une poignée (1a, 6a, 8a) est disposée sur ledit élément de corps à proximité de ladite seconde extrémité.
